Europäisches Patentamt

European Patent Office

Office européen des brevets

(11) Publication number: **0 489 445 A2**

# EUROPEAN PATENT APPLICATION

(21) Application number: **91120998.9**

(51) Int. Cl.⁵: **G01N 1/18, G01N 30/80**

(22) Date of filing: **06.12.91**

(30) Priority: **06.12.90 JP 405338/90**

(43) Date of publication of application:
**10.06.92 Bulletin 92/24**

(84) Designated Contracting States:
**CH DE FR GB IT LI NL**

(71) Applicant: **SUMITOMO ELECTRIC INDUSTRIES, LTD**
**5-33, Kitahama 4-chome, Chuo-ku**
**Osaka-shi, Osaka-fu(JP)**

(72) Inventor: **Kishimoto, Toshihiko, c/o Osaka Works of Sumitomo**
**El.Ind.Ltd., 1-3, Shimaya 1-chome, Konohana-ku**
**Osaka-shi, Osaka-fu(JP)**
Inventor: **Niwa, Shinichiro, c/o Osaka Works of Sumitomo**
**El.Ind.Ltd., 1-3, Shimaya 1-chome, Konohana-ku**
**Osaka-shi, Osaka-fu(JP)**
Inventor: **Nakabayashi, Makoto, c/o Osaka Works of Sumitomo**
**El.Ind.Ltd., 1-3, Shimaya 1-chome, Konohana-ku**
**Osaka-shi, Osaka-fu(JP)**
Inventor: **Umemoto, Misako, c/o Osaka Works of Sumitomo**
**El.Ind.Ltd., 1-3, Shimaya 1-chome, Konohana-ku**
**Osaka-shi, Osaka-fu(JP)**
Inventor: **Uno, Atsushi c/o Osaka Works of Sumitomo**
**El.Ind.Ltd., 1-3, Shimaya 1-chome, Konohana-ku**
**Osaka-shi, Osaka-fu(JP)**

(74) Representative: **Lehn, Werner, Dipl.-Ing. et al**
**Hoffmann, Eitle & Partner Patentanwälte**
**Arabellastrasse 4**
**W-8000 München 81(DE)**

(54) **Method and apparatus for fractionation.**

(57) A material is effectively fractionated from a liquid sample by flowing out a solution containing the material which is separated from a liquid sample from an outlet (3) while relatively moving the outlet (3) and a spreading body (4) which is placed facing the outlet to spread the solution containing the separated material on the spreading body (4).

Fig. 2

## BACKGROUND OF THE INVENTION

### Field of the Invention

The present invention relates to a method and an apparatus for fractionation. More particularly, the present invention relates to a method and an apparatus for fractionation which achieves the fractionation of a material not only in a conventional order of 10 $\mu$l or 10 $\mu$g but also in a much smaller order of nanogram.

In particular, the present invention is suitable for microanalysis or purification of biomaterials such as proteins.

### Description of the Related Art

Hitherto, to separate a desired material from a liquid sample containing plural materials, liquid chromatography has been used. The liquid chromatography comprises flowing a moving phase such as an eluent through an column which is filled with a fixed phase such as a porous gel, and injecting a sample to be fractionated containing plural solutes to separate the solutes during the sample flows through the column. The chromatography includes gel permeation chromatography (GPC), ion exchange chromatography, hydrophobic chromatography, chromatofocusing, affinity chromatography, partition chromatography, and the like.

Recently, high performance liquid chromatography is widely used for analysis or large scale separation, which chromatography comprises filling a finely divided homogenized chromatographic agent in a pressure column and flowing a liquid to be analyzed under high pressure to shorten the fractionation time greatly.

The liquid chromatography is classified according to fractionation modes into (a) chromatography in which a solute (material) is separated according to its size and (b) chromatography which utilizes an interaction between the solute and the solid phase. For example, GPC utilizes a column filled with porous particles and separates the solutes using micropores or networks in the particles. The solutes are separated based on the difference of its size (a size of a molecule).

The ion exchange chromatography utilizes an ionic interaction between ionic sites of the solute and ion-exchange groups of a solid phase and is suitable for separation of proteins based on the difference of the electrical properties, in particular, for purification of an enzyme. The affinity chromatography utilizes a specific affinity of a solute to a solid phase and includes a chromatography utilizing formation of a specific complex of an enzyme and a substrate and immune affinity chromatography utilizing an antigen-antibody reaction.

In the liquid chromatography, a molecular sieve (for example, a porous gel), an ion-exchanging agent or an affinity chromatographic agent is filled in a column and then a sample is placed on the top of the column with supplying an eluent from the top of the column, or a mixture of the sample and the eluent is supplied from the bottom of the column. Each component in the sample is separated during the sample flows through the column, and the component is separately collected in one of plural containers such as test tubes in a fraction collector in the eluting sequence. A concentration of the solute such as the protein in each fraction which flows out from a solution outlet of the column can be measured by, for example, a UV monitor.

Another method for separation of a desired material in liquid sample containing plural materials comprises adding the sample in an centrifugation tube in which a density gradient is formed with, for example, sucrose and the sample is centrifugally separated at a revolution rate of 1000 to 80,000 rpm to separate the desired material based on difference of a density between the materials. The desired material is recovered by collecting the solution at a predetermined depth in the tube with a syringe through a syringe needle.

In the above conventional separation methods, the minimum volume of the sample to be separated is in the order of several ten $\mu$l to ml. Further, the solute is collected in the form of a solution, it is highly possible that once separated solute are remixed with other solute in the test tube or the syringe.

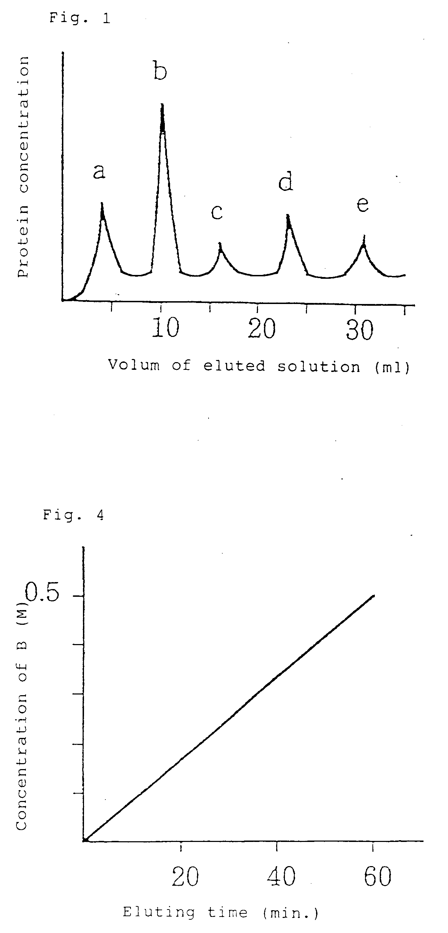

For example, Fig. 1 shows a separation pattern of proteins by column chromatography, in which a concentration of an eluted protein is read by the UV monitor. In Fig. 1, the ordinate represents the protein concentration and the abscissa represents an eluted volume. Each peak position corresponds to a kind of each protein. Eluted liquids are collected in plural test tubes of a fraction collector according to the eluting sequence. In the separation pattern of Fig. 1, when the first 5 ml is collected in a test tube, only a half amount of the protein corresponding to the peak (a) is recovered. Then, the next amount from 5 ml to 10 ml is collected in a next test tube, a remaining half amount of the protein corresponding to the peak (a) and a half amount of the protein corresponding to the peak (b) are recovered. Accordingly, the once separated proteins are mixed when the fraction is recovered in the test tube, and the desired material cannot be separated with accurate correspondence to the separation pattern.

In particular, when an amount of the desired material is very small or when the sample contains two or more materials molecular sizes or the interactions with the solid phase of which are similar to each other, the desired material cannot be precisely fractionated.

It may be contemplated to recover the eluted fraction drop by drop. But, if the fraction amount is minute, for example, in the order of $\mu l$, it is difficult to form a droplet. Further, when the fraction is recovered in the form of a droplet, remixing of the separated materials cannot be avoided.

## SUMMARY OF THE INVENTION

An object of the present invention is to provide a method and an apparatus for precisely fractionating a material corresponding to a separation pattern when the desired material is separated from a liquid sample containing plural materials.

Another object of the present invention is to provide a method and an apparatus for precisely fractionating a very small amount material from a liquid sample.

According to a first aspect of the present invention, there is provided a method for fractionating a material from a liquid sample, which comprises flowing out a solution containing a material which is separated from a liquid sample from an outlet while relatively moving said outlet and a spreading body which is placed facing said outlet to spread said solution containing said separated material on said spreading body.

According to a second aspect of the present invention, there is provided an apparatus for fractionating a material from a liquid sample, which comprises an outlet from which a solution containing a material which is separated from a liquid sample, a spreading body on which said solution is spread and which faces said outlet, and means for relatively moving said outlet and said spreading body.

## BRIEF DESCRIPTION OF THE DRAWINGS

Fig. 1 shows a separation pattern of proteins,
Figs. 2 and 3 show examples of the method and apparatus of the present invention, in which liquid chromatography is used for the separation of the desired material from the liquid sample,
Figs 4 and 5 show the eluting conditions used in Examples 1 and 2, respectively.

## DETAILED DESCRIPTION OF THE DRAWINGS

According to the present invention, the solution containing material which is separated from the liquid sample by, for example, liquid chromatography is flowed out from an outlet of, for example, a chromatography column and then spread on the spreading body which is placed facing to the outlet while the outlet and the spreading body are moved relatively to each other, whereby the separation pattern is closely reproduced on the surface of the spreading body.

In the method of the present invention, by adjusting a flow out rate of the fraction from the outlet and/or the relative speed of the outlet and the spreading body, a minor amount material or plural materials having close molecular sizes, which may not be separated by the conventional methods, can be easily separated.

Since the materials which are separated can be successively fixed on the spreading body by the method of the present invention, the remixing of the separated materials is avoided, and the materials are easily separated, purified, analyzed or fractionated. In the conventional technique, for example, when a protein is to be fractionated, a protein fraction should be subjected to amino acid analysis after desalting and freeze dried. But, according to the present invention, the protein fraction which is spread on the spreading body can be directly subjected to the amino acid analysis,

For separating the desired material from the liquid sample containing plural materials, there are used liquid chromatography (e.g. GPC, ion exchange chromatography, hydrophobic chromatography, chromatofocusing, affinity chromatography, partition chromatography, etc.), centrifugal separation and electrophoresis. Among them, liquid chromatography using a column is preferred in view of easy spreading of the solution.

A material to be separated is not limited. Preferably, a material is unvolatile at room temperature. Among the materials, biomaterials such as proteins (e.g. enzymes) are preferably analyzed, fractionated or purified by the method of the present invention.

The spreading body on which the separated material is spread is a plane body and may be made of any material which can hold the eluted liquid such as a water-absorbing material, a hydrophobic material and a porous material.

Specific examples of the spreading body are membranes of natural or synthetic polymers such as polyamide, nitrocellulose, polytetrafluoroethylene, polyethylene and the like. A membrane of an organic polymer which is chemically treated and modified (e.g. nitrocellulose) is also usable.

Also, as the spreading body, membranes of inorganic polymers such as graphite, porous glass or silica; metal membranes of aluminum or apatite; ceramic membranes of alumina or silicon nitride; and crystalline salt may be used. Their surfaces nay be chemically or physically modified.

Further, as the spreading body, a plane member which carries thereon organic, inorganic, metal or ceramic particles can be used. Examples of the organic particles are those of polyamide, nitrocellulose, cellulose, polytetrafluoroethylene, polyethylene and the like. Examples of the inorganic particles are those of graphite, porous glass, silica and the like. Examples of the metal particles are those of aluminum, apatite and the like. Examples of the ceramic particles are those of alumina and the like. The organic particles may be chemically modified by, for example, hydrolysis or physically modified by, for example, plasma irradiation. The inorganic, metal or ceramic particles may be physically modified by, for example, ion plating or chemically modified.

In addition, as the spreading body, a gel-form one such as agarose gel, polyacrylamide gel or various films containing an impregnated solution may be used. Besides the gel-form one, those in a dry state or in a highly viscous state may be used.

For spreading the solution containing the separated material on the spreading body, the outlet and the spreading body are placed to face each other and relatively moved while flowing out the solution from the outlet, whereby the solution is spread on the surface of the spreading body.

For example, when liquid chromatography is used for the separation, the eluates each containing the respective separated material flow out from the outlet in the eluting sequence of the eluates as time passes and then the eluates are spread on the surface of the spreading body.

In this case, the relative movement of the outlet and the spreading body can be effected by various methods as follows:

(1) The solution outlet, for example, a nozzle is fixed, while the spreading body to which the outlet is contacted is horizontally moved to spread the solution on the surface of the spreading body.

(2) The nozzle is contacted to the surface of the film-form spreading body which is wound around a roll.

(3) The nozzle is contacted to and moved over the surface of the spreading body in various directions.

(4) The disc-shape spreading body is rotated while a tip end of the nozzle is contacted to the surface of the spreading body and moved gradually inward or outward.

Two or more of the above methods may be combined.

The present invention is not limited to the above moving methods, insofar as the solution outlet and the spreading body are relatively moved and the solution is spread on the surface of the spreading body. When the highly viscous spreading body is used, the tip end of the outlet is inserted in the spreading body and the solution can be retained therein.

## PREFERRED EMBODIMENTS OF THE INVENTION

The present invention will be explained further in detail by making reference to the accompanying drawings.

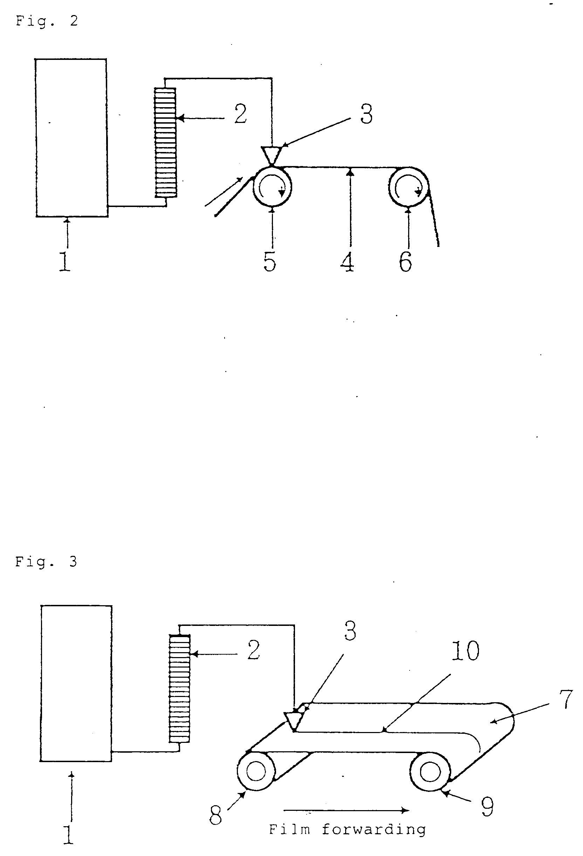

Figs. 2 and 3 show examples in which liquid chromatography is used for the separation of the desired material from the liquid sample.

In Fig. 2, the sample is poured in a high performance liquid chromatography (HPLC) apparatus 1. Eluates each containing a material which has been separated in a column 2 flow out from a nozzle 3 and are spread on a surface of a spreading membrane 4 in the eluting sequence. The membrane 4 is horizontally moved by means of a pair of rolls 5 and 6. A tip end of the nozzle 3 is separated from the membrane surface at such distance that the eluate is not fractionated in the form of droplet or is contacted to the membrane surface. When the flow rate of the eluates and the moving rate of the membrane are sufficiently large, the tip end of the nozzle is not necessarily contacted to the membrane surface. To precisely fractionate a minor material, the eluate is preferably spread on the membrane surface with the tip end of the nozzle being very close to the membrane surface or being contacted to the membrane surface in order to avoid the fractionation in the droplet form.

In Fig. 3, a membrane 7 is unwound from a roll 8 and the eluate 10 is linearly spread on the membrane 7. Then, the membrane is wound around a roll 9.

Since the eluates are spread on the membrane surface in the eluting sequence as time passes, the materials which are separated by HPLC can closely reproduce the separation pattern of the materials produced by the separation by HPLC when the eluting rates of the solutions and/or the relative speed between the nozzle 3 and the spreading body are suitable adjusted.

The outlet is not necessarily fixed and may be moved right and left, or right, left, back and forth. When the spreading body is in the disc form, the outlet can be moved inwardly or outwardly while the disc body is rotated, Such moving means may be suitably selected by those skilled in the art.

The eluting rates of the solutions and the relative speed between the outlet and the spreading body depend on the material of the spreading body, desired accuracy of the fractionation, etc. The eluting rate of the solution is generally from 0.001 $\mu$l/min. to 10 ml/min. in view of the possible analyzable minimum amount, material amounts and an amount of a sample in a usual analysis, prefer-

ably from 0.01 μ 1/min. to 5 ml/min. in view of an amount treated in usual column chromatography, more preferably from 0.01 μl/min. to 1 ml/min. in view of an amount treated in HPLC. To accurately fractionate the minor materials which are separated adjacently, the relative rate of the outlet and the spreading body is usually from 10 μm/min. to 7000 cm/min., preferably from 100 μm/min. to 1000 cm/min., more preferably from 1 mm/min. to 100 cm/min.

A ratio (A) of the eluting rate (ml/min.) to the relative rate (cm/min.) is usually from $10^{-10}$ to $10^4$, preferably from $10^{-7}$ to $10^3$, more preferably from $10^{-5}$ to 1000.

The present invention will be illustrated by following Examples.

Example 1

Lysozyme Protein (distributed by Yashima Chemical Co., Ltd.) was separated by HPLC (manufactured by TOSOH) under the below described conditions. The separated sample was spread on a nitrocellulose membrane under the below described conditions.

(1) Lysozyme Protein was dissolved in 25 mM phosphate buffer (pH 6.4) in a concentration of 1 mg/ml (hereinafter referred to as "Sample").

Fractionation was carried out using, as a gel column, TKSgel CM-3SW made of stainless steel having an inner diameter of 4.5 mm and a length of 7.5 cm by injecting 100 μl of the Sample.

Eluent

A:     25 mM phosphate buffer (pH 6.4)
B:     A + 0.5 M NaCl

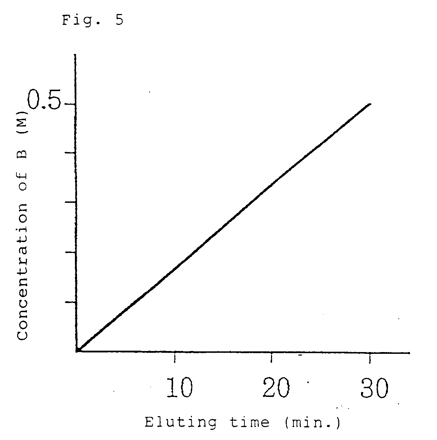

Eluates were eluted at a flow rate of 0.3 ml/min. by a linear gradient elution such that a gradient of the NaCl concentration changed from 0 to 0.5 M in a period of 60 minutes as shown in Fig. 4.

The eluate containing each component which was separated in the column was spread on the nitrocellulose membrane (manufactured by Amashamu) which horizontally moved at a rate of 1.5 cm/min. That is, as shown in Fig. 2, the eluate containing the material which was separated in the column 2 was spread on the nitrocellulose membrane 4 having a width of 5 cm which horizontally moved with contacting the nozzle 3 to the membrane surface. The rolls 5 and 6 were rotated at a peripheral velocity of 1.5 cm/min. A distance between the rolls 5 and 6 was about 30 cm.

The nitrocellulose membrane was beforehand wetted with a mixture of 10 mM Tris-HCl (pH 8.0) and 0.15 M NaCl and used in a wet state. During the operation, the membrane was wetted by spraying water to prevent drying.

(2) After spreading the eluate on the membrane, a filter paper was placed on the membrane to remove excessive water, and the membrane was dipped in a mixture 200 ml of 10 mM Tris-HCl (pH 8.0), 0.15 M NaCl and 0.05 % by weight of Tween 20 (TBST) and washed.

(3) Then, the membrane was dipped in a mixture of 10 mM of Tris-HCl (pH 8.0), 0.15 M NaCl, 0.5 % by weight of Tween 20 (TBST) and 0.1 % by weight of BSA for 30 minutes.

(4) The membrane was rinsed with TBST three times.

(5) Thereafter, the membrane was dipped in a 1 μg/ml solution of an anti-lysozyme antibody (purified from a supernatant of incubated mouse hybridoma) in TBST for 30 minutes.

(6) The membrane was rinsed with TBST three times.

(7) The membrane was dipped in a 1 μg/ml solution of an alkali phosphatase-bound anti-mouse IgG antibody in TBST for 30 minutes.

(8) The membrane was rinsed with TBST five times.

(9) After the membrane was dipped in a mixture of 100 mM Tris-HCl (pH 9.5), 100 mM NaCl and 5 mM $MgCl_2$, NBT and BCIP were added to the mixture till their final concentrations reached 5 mM and 5 mM, respectively, followed by keeping the mixture containing the membrane in a dark place for one hour.

(10) A part of the membrane where lysozyme was eluted was turned purple, and the position corresponded to 22-24.5 minutes.

(11) As described above, the eluate was spread on the membrane and the protein was fractionated.

Example 2

As a protein sample solution, a mixture of myoglobin, α-chymotrypsinogen, cytochrome C and lysozyme (each 1 mg/ml) in 20 mM phosphate buffer (pH 7.4) was used, and an each protein was analyzed as follows:

(1) Separation conditions

HPLC system (manufactured by TOSOH) was used.

Gel column

TSKgel CM-35W (stainless steel made)
Inner diameter of 2.1 mm and length of 10 cm

Eluate

A: 25 mM phosphate buffer (pH 7.4)

B: A + 0.5 M NaCl

Eluates were eluted at a flow rate of 0.2 ml/min. by a linear gradient elution such that a gradient of the NaCl concentration changed from 0 to 0.5 M in a period of 30 minutes as shown in Fig. 5.

The above proteins mixture (10 $\mu$l) was injected in the HPLC system and eluted under the above conditions.

The outlet of the eluates was contacted to the nitrocellulose membrane (manufactured by Amashamu) which horizontally moved at a rate of 10 cm/min.

The nitrocellulose membrane was beforehand wetted with a mixture of 10 mM Tris-HCl (pH 8.0) and 0.15 NaCl and used in a wet state.

By the above procedures, each protein which was separated in the HPLC was spread on the membrane (Fig. 2).

(2) The same steps as the steps (2) to (9) of Example 1 were repeated.

(3) The color development due to lysozyme was found at a position corresponding to 19.8 to 20 minutes.

(4) In the same manner as in the step (5) of Example 1 but using an antibody against $\alpha$-chymotrypsinogen A in place of the anti-lysozyme antibody, the procedure was carried out. Thereafter, the step (4) and the subsequent steps of Example 1 were repeated.

(5) The color development due to $\alpha$-chymotrypsinogen was found at a position corresponding to 14.3 to 14.5 minutes.

(6) With cytochrome C, the same procedures were repeated, and the color development was found at a position corresponding to 14.55 to 14.75 minutes.

As above, the three proteins were clearly separated. Since the two components, namely $\alpha$-chymotrypsinogen and cytochrome C were contained in 80 $\mu$l, they are not fractionated by the conventional methods, but can be by the present invention.

**Claims**

1. A method for fractionating a material from a liquid sample, which comprises flowing out a solution containing a material which is separated from a liquid sample from an outlet while relatively moving said outlet and a spreading body which is placed facing said outlet to spread said solution containing said separated material on said spreading body.

2. The method according to claim 1, wherein a flow rate of said solution if from 0.001 $\mu$l/min.

to 10 ml/min., and a relative moving rate of said outlet and said spreading body is from 0.001 to 7000 cm/min.

3. The method according to claim 1, wherein a ratio of a flow rate (ml/min.) of said solution to a relative moving rate (cm/min.) of said outlet and said spreading body is from $10^{-10}$ to $10^4$.

4. The method according to claim 1, wherein said spreading body is one selected from the group consisting of natural and synthetic polymer membranes and inorganic polymer membranes.

5. The method according to claim 1, wherein said spreading body comprises particles of a natural or synthetic polymer or an inorganic polymer.

6. The method according to claim 1, wherein said spreading body is in a gel form.

7. The method according to claim 1, wherein said solution is continuously spread on said spreading body.

8. The method according to claim 1, wherein a tip end of said outlet and a surface of said spreading body is uncontacted.

9. The method according to claim 1, wherein a tip end of said outlet and a surface of said spreading body is contacted.

10. The method according to claim 1, wherein said solution contains a material which is separated by liquid chromatography.

11. An apparatus for fractionating a material from a liquid sample, which comprises an outlet from which a solution containing a material which is separated from a liquid sample, a spreading body on which said solution is spread and which faces said outlet, and means for relatively moving said outlet and said spreading body.

Fig. 1

Volum of eluted solution (ml)

Fig. 4

Eluting time (min.)

Fig. 2

Fig. 3

Film forwarding

Fig. 5